# EUROPEAN PATENT SPECIFICATION

(11) **EP 1 185 866 B1**
(45) Date of publication and mention of the grant of the patent: **11.02.2009**
(21) Application number: 00930888.3
(22) Date of filing: 07.06.2000
(51) Int. Cl.: G01N 33/53, G01N 33/68, G01N 33/574

(54) **A METHOD OF DETERMINING POTENTIAL SUSCEPTIBILITY TO DEVELOPMENT OF ALTE AND/OR SIDS**
VERFAHREN ZUM BESTIMMEN DER POTENZIELLEN EMPFINDLICHKEIT FüR DIE ENTWICKLUNG VON ALTE UND/ODER SIDS
PROCEDE DE DETERMINATION DE LA SUSCEPTIBILITE POTENTIELLE DE DEVELOPPER DES EPISODES VISIBLES A RISQUE MORTEL (ALTE) ET/OU LE SYNDROME DE LA MORT SUBITE DU NOURRISSON (MSN)

(30) Priority: 07.06.1999 AU PQ081099
(43) Date of publication of application: 13.03.2002
(73) Proprietor: Newcastle Innovation Limited, Callaghan, NSW 2308 (AU)
(72) Inventor: CLANCY, Robert, Newcastle, NSW 2300 (AU); GLEESON, Maree, Merewether, NSW 2291 (AU)
(74) Representative: Wakerley, Helen Rachael
(86) International application number: PCT/AU2000/000643
(87) International publication number: WO 2000/075656

(56) References cited:
- EP-A- 0 139 201
- US-A- 5 556 759
- US-A- 5 556 759
- US-A- 5 747 266
- SIARAKAS S ET AL: "Immunological evidence for a bacterial toxin aetiology in sudden infant death syndrome." FEMS IMMUNOLOGY AND MEDICAL MICROBIOLOGY. NETHERLANDS 1 AUG 1999, vol. 25, no. 1-2, 1 August 1999 (1999-08-01), pages 37-50, XP001118074 ISSN: 0928-8244
- FORSYTH K D ET AL: "Lung immunoglobulins in the sudden infant death syndrome." BMJ (CLINICAL RESEARCH ED.) ENGLAND 7 JAN 1989, vol. 298, no. 6665, 7 January 1989 (1989-01-07), pages 23-26, XP001068238 ISSN: 0959-8138
- BAXENDINE J A ET AL: "PULMONARY EOSINOPHILIA IN SUDDEN INFANT DEATH SYNDROME" JOURNAL OF PATHOLOGY, CHICHESTER, SUSSEX, GB, no. 177, December 1995 (1995-12), pages 415-421, XP001068221 ISSN: 0022-3417
- STOLTENBERG L ET AL: "CHANGES IN THE CONCENTRATION AND DISTRIBUTION OF IMMUNOGLOBULIN-PRODUCING CELLS IN SIDS PALATINE TONSILS" PEDIATRIC ALLERGY AND IMMUNOLOGY, COPENHAGEN, DK, no. 6, February 1995 (1995-02), pages 48-55, XP001068223 ISSN: 0905-6157
- GLEESON M ET AL: "MUCOSAL IMMUNE RESPONSE IN A CASE OF SUDDEN INFANT DEATH SYNDROME" PEDIATRIC RESEARCH, WILLIAMS AND WILKINS, BALTIMORE, MD,, US, vol. 6, no. 33, 1993, pages 554-556, XP001077828 ISSN: 0031-3998
- VEGE A ET AL: "IL-6 cerebrospinal fluid levels are related to laryngeal IgA and epithelial HLA-DR response in sudden infant death syndrome." PEDIATRIC RESEARCH. UNITED STATES JUN 1999, vol. 45, no. 6, June 1999 (1999-06), pages 803-809, XP001117828 ISSN: 0031-3998
- M. GLEESON ET AL.: 'Mucosal immune response in a case of sudden infant death syndrome' PEDIATRIC RESEARCH vol. 33, no. 6, 1993, pages 554 - 556, XP001077828
- K.D. FORSYTH ET AL.: 'Lung immunoglobulins in the sudden infant death syndrome' BMJ vol. 298, January 1998, pages 23 - 26, XP001068238
- J.A. BAXENDINE AND I.E. MOORE: 'Pulmonary eosinophilia in sudden infant death syndrome' JOURNAL OF PATHOLOGY vol. 177, 1995, pages 415 - 421, XP001068221
- P.S. THRANE ET AL.: 'Increased immune response in upper respiratory and digestive tracts in SIDS' THE LANCET vol. 335, no. 8683, 27 January 1990, pages 229 - 230, XP001064747
- L. STOLTENBERG ET AL.: 'Sudden infant death syndrome victims show local immunoglobulin M response in tracheal wall and immunoglobulin A response in duodenal mucosa' PEDIATRIC RESEARCH vol. 31, no. 4, 1992, pages 372 - 375, XP001068229
- L. STOLTENBERG ET AL.: 'Changes in the concentration and distribution of immunoglobulin-producing cells in SIDS palatine tonsils' PEDIATR. ALLERGY IMMUNOL. vol. 6, 1995, pages 48 - 55, XP001068223
- G.E.D. URQUHART ET AL.: 'Sudden unexplained death in infancy and hyperimmunization' J. CLIN. PATH. vol. 24, 1971, pages 736 - 739, XP001068236
- DANIEL P. STITES AND ABBA I. TERR: 'The mucosal immune system', 1991, APPLETON & LANGE (A DIVISION OF PRENTICE-HALL INTERNATIONAL INC.) XP002953308 Basic and Clinical Immunology, Seventh Edition, Chapter 15 * page 175 - page 186 *
- M. GLEESON ET AL.: 'The variability of immunoglobulins and albumin in salivary secretions of children' SCAND. J. IMMUNOL. vol. 33, no. 5, 1991, pages 533 - 541, XP001068237
- J.G. BROOKS: 'Apparent life-threatening events and apnea of infancy' CLINICS IN PERINATOLOGY vol. 19, no. 4, 1992, pages 809 - 838, XP001076627
- D.C. SHANNON: 'Prospective identification of the risk of SIDS' CLINICS IN PERINATOLOGY vol. 19, no. 4, 1992, pages 861 - 869, XP001068234

## Description

### TECHNICAL FIELD

The present invention relates to methods for determining predisposition to acute life threatening episodes (ALTE) and/or sudden infant death syndrome (SIDS) and in particular to methods of assessing potential susceptibility to development of ALTE and/or SIDS by determining a subject's total IgA and/or IgA1.

### BACKGROUND

A great deal has been done to minimize the risk of SIDS by non-specific methods related to infant care. However, prevention using specific assays related to causal mechanisms has not been explored. Identifying a causal mechanism may be expected to make a major impact on SIDS outcome through general awareness, and if used in conjunction with non-specific nursing care. The development of new techniques for identifying infants at risk of SIDS could be a significant outcome.

Interest in this approach to the prevention of SIDS arose as a result of an unusual opportunity of observing a 'prospective' case of SIDS during a study of 250 normal infants [1]. The infants were followed from birth, measuring parameters of immune status in saliva. The key observation in the one child who died from SIDS was an extraordinarily high IgM level appearing after a mild respiratory tract infection, several weeks before the child suddenly died. While all parameters tested in the SIDS victim (i.e. albumin, IgG and IgA) were in excess of the 90th percentile level, relative levels of IgM were the highest, being more than three times the level of the 90th percentile figure (compared to approximately one and a half times the 90th percentile level for albumin, IgG and IgA). This observation in a single case was consistent with post mortem studies showing large numbers of 1gM containing plasma cells in the trachea and gut of subjects dying from SIDS [2-5]. The level of IgM was much in excess of any small increases seen in matched infection control studies [6]. These observations raised the possibility that assay of IgM in saliva of infants with an upper respiratory tract infection may be a very useful marker of risk of SIDS, reflecting the disturbed mucosal immunoregulation that underpins the risk.

The numerous epidemiological studies of SIDS have identified many of the risk factors of SIDS but have failed to find a cause [7]. The role of infection and disturbed immunity has been proposed as one of the potential mechanisms for SIDS [8]. The common findings at autopsy of SIDS infants are consistent with infection or inflammation as a contributing cause of death [9]. SIDS has been reported to occur after a mild upper respiratory tract infection (URTI) [9-12], however there is no evidence that favours infections by any virulent pathogen. A low grade pathogen, that results in overstimulation of the immune system may be one important link in the chain of events that culminates in respiratory arrest.

There is evidence from post mortem studies [2-5, 13-14] and a prospective case study [1] of a gross disturbance of mucosal immunity in SIDS associated with prior respiratory illness or inflammation. These studies suggest an infective agent is responsible for the disturbance observed in the immune parameters which thus provides a clinical "trigger" for testing the infant for risk..

Infants presenting with episodes of apnoea from which the infant recovers are termed acute life threatening episodes (ALTEs) and are classified as "near-miss" SIDS when no underlying medical condition is identified. ALTE children could, therefore, be expected to have a similar pattern of dysregulation of mucosal immunity to SIDS children.

To date, however, no method exists by which a prediction of the potential susceptibility to development of ALTE and/or SIDS can be carried out on the basis of a specific immunological assay.

It is an object of the present invention to overcome or ameliorate at least one of the disadvantages of the prior art, or to provide a useful alternative.

### SUMMARY OF THE INVENTION

As indicated above, the prior art [1] pointed towards IgM levels after URTI infection as being a potentially useful parameter for study in ALTE/SIDS research. However, it has been unexpectedly found that IgA levels were significantly and consistently higher in ALTE or "near miss" SIDS cases. IgA can therefore be used as a predictor of susceptibility to development of ALTE and/or SIDS.

According to a first aspect, the present invention provides a method of assessing potential susceptibility to development of ALTE and/or SIDS in a subject including:
a) determination of the immuoglogulin A (IgA) level in a sample from the subject; and
b) prediction of susceptibility to development of ALTE and/or SIDS by comparison of said IgA level with a predetermined standard.

According to a second aspect, the present invention provides a method of assessing potential susceptibility to development of ALTE and/or SIDS in a subject including:
a) determination of immunoglobulin A1 I (lgA1) level in a sample from the subject; and
b) prediction of susceptibility to development of ALTE and/or SIDS by comparison of said IgA1 level with a predetermined standard.

Preferably, the subject is a human infant.

Preferably; the sample is a sample from a subject at the time of, or any time up to approximately 2 weeks after, an upper respiratory tract infection (URT1) or symptoms.

Preferably, the immunoglobulin is secretory immunoglobulin. Preferably, the secretory immunoglobulin is salivary immunoglobulin. Preferably, the sample is whole unstimulated saliva. However, it will be clear to the skilled addressee that other body secretions known to contain IgA and/or IgA 1 would also be useful as samples for the present method.

Preferably, the subject is not fasting when the sample is collected.

Preferably, the immunoglobulin level is determined by ELISA. However, it will be understood that the immunoglobulin level may be determined by radial immunodiffusion and/or similar methods, all of which would be known to a skilled addressee. The method is particularly suitable for an assay in which the immunoglobulin level is analysed by a rapid, near-subject assay. It can thus provide a yes/no test for immediate action.

In one embodiment, the immunoglobulin level is determined by contacting a body secretion with an assay device or system on a support. The sample need not necessarily be removed from the subject but the method may be applied *in situ.* For example, the immunoglobulin level may be analysed by contacting an assay device or system with the saliva of the subject *in situ.*

The predetermined standard may be any appropriate standard, for example, a normal population standard or an internal personal standard. The skilled addressee will recognize the types of standards which will be useful in the present invention.

It will be clear to the skilled addressee that the determination of the level of IgA or IgA1 could also be used as a predictor of susceptibility to development of ALTE and/or SIDS in conjunction with other indices such as other immunoglobulins, for example IgM or IgG, acute phase reactants or cellular components.

In the context of the present invention, the word "standard" includes within its meaning, but is not limited to, the normal population level of immunoglobulin ie. the average IgA or IgA1 value for age-matched normal subjects. It may also be an internal personal standard i.e. the level of IgA or IgA1 in a sample taken previously from the same individual.

### BRIEF DESCRIPTION OF THE FIGURES

- Figure 1: IgA concentration levels (mg/L) for ALTE (■), "mild" (O) and well (▲) infants - initial sample.
- Figure 2: 1gM concentration levels (mg/L) for ALTE (■), "mild" (O) and well (▲) infants - initial sample.

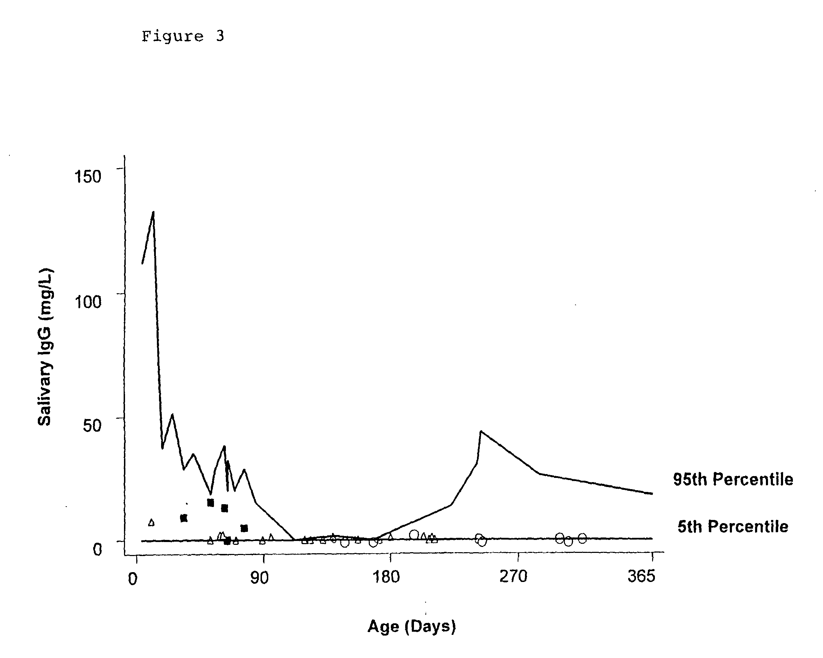

- Figure 3: IgG concentration levels (mg/L) for ALTE (■), "mild" (O) and well (▲) infants - initial sample.

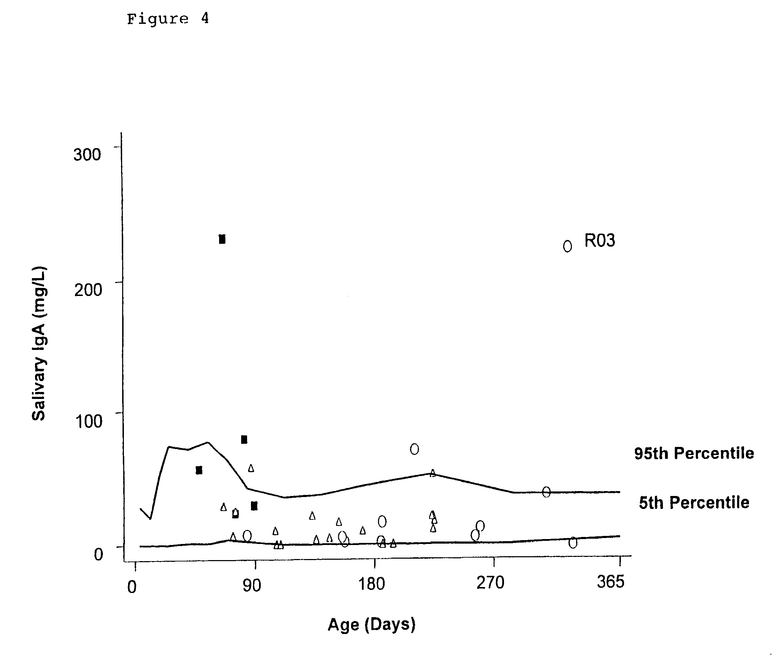

- Figure 4: IgA concentration levels (mg/L) for ALTE (■), "mild" (O) and well (▲) infants - 14 day sample. Subject RO3 was assessed 12 days post infection.

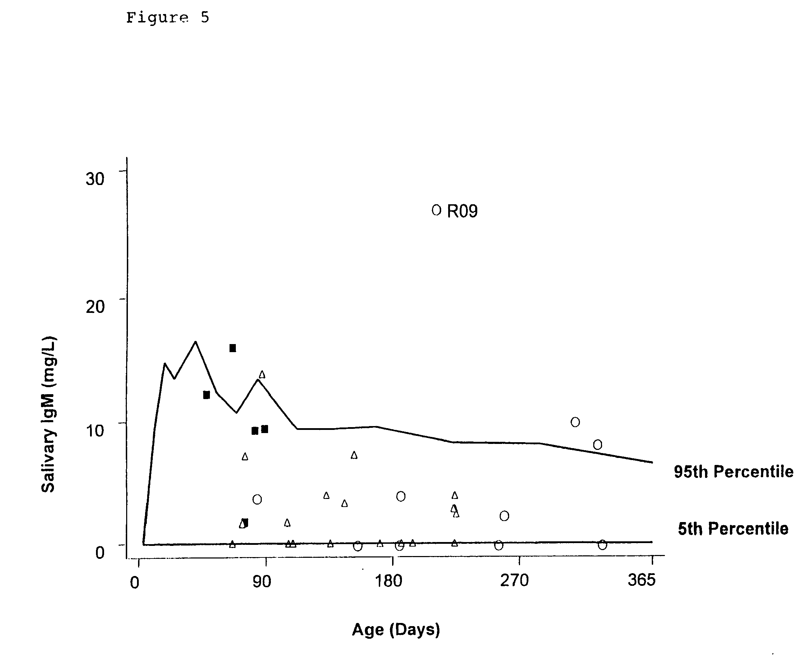

- Figure 5: IgM concentration levels (mg/L) for ALTE (■), "mild" (O) and well (▲) infants - 14 day sample. Subject RO9 was assessed 14 days post immunisation with triple antigen and *Haemophilus influenzae B.*

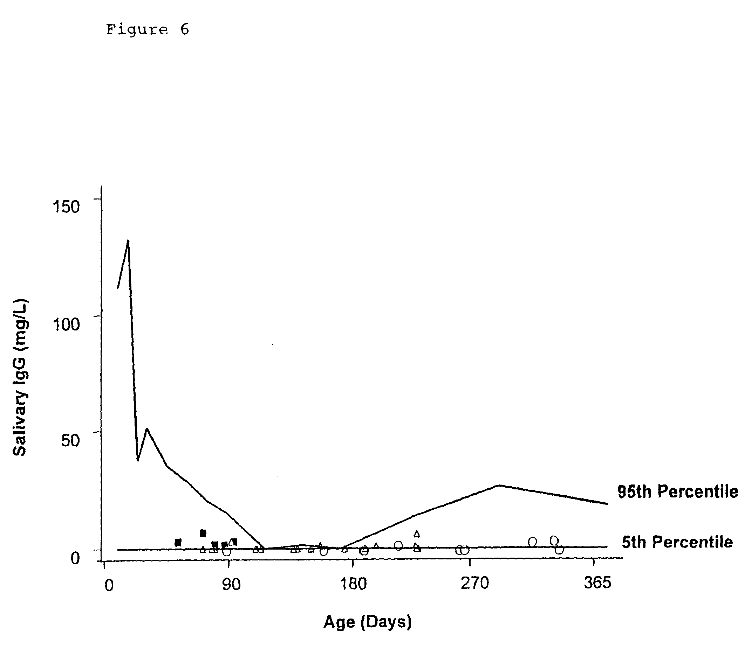

- Figure 6: IgG concentration levels (mg/L) for ALTE (■), "mild" (O) and well (▲) infants - 14 day sample.
- Figure 7A: IgA1 concentration levels (mg/L) for ALTE, "mild" and well infants.
- Figure 7B: Data of Figure 7A shown with 95% confidence intervals.
- Figure 8: Total IgA concentration levels (mg/L) for ALTE, "mild" and well infants in which IgA1 concentration levels (mg/L) is shown in Figures 7A and 7B.

### DESCRIPTION OF THE INVENTION

A preferred embodiment of the invention will now be described, by way of example only.

### EXAMPLE 1

### Total IgA, IgG and IgM levels in Saliva of Infants with ALTE

### Saliva Collection

Whole mixed saliva was collected by gentle suction from the buccal cavity of the mouth [15]. This technique is successful in children (aged from 1 day) and adults [1,16].

### Questionnaire

A standardised questionnaire was used to collect the relevant SIDS related demographics. The classification into the "near-miss'' SIDS group (ALTE) was made by the attending paediatrician on the basis of clinical investigations.

### Saliva Tests

Salivary immunoglobulins were measured by ELISA and albumin by rate nephelometry (Beckman, ARRAY) [16].

### Statistical Analysis

The differences in mucosal immune parameters was determined between the ALTE infants and two control groups of subjects (mild URTI and well infants) using analysis of variance (ANOVA) or the appropriate non-parametric statistics.

### Subjects

37 subjects aged 1-10 months were recruited (20 males, 17 female) in 3 categories:
- Acute life Threatening episodes (ALTE) at John Hunter Hospital (n=5)
- Mild respiratory tract illness (MILD) from General Practitioners (n=11)
- A well control group (WELL) from immunisation clinics (n=21).

### Questionnaire Data

• There were more males (n=4) than females (n=1) in the ALTE group.
• There were no significant differences between the groups for age, birth history, family demographics, ethnic background or family history of SIDS.
• There were a higher percentage of children exposed to passive tobacco smoke (60%, n=3) in the ALTE group compared to the MILD (36%, n=4) and WELL (10%, n=2) control groups (p=0.03).
• The ALTE group had a higher percentage of families in the average-below average socio-economic category (100%) compared to the other control groups (p<0.01).
• There were no differences between the groups for feeding history, immunisation status, sleeping position.
• In 4 of the 5 ALTE subjects an Upper Respiratory Tract Illness (URTI) was suspected as the cause of the ALTE (Table 1).

| TABLE 1 | | | | | | | |
|---|---|---|---|---|---|---|---|
| Doctor Questionnaire - ALTE | | | | | | | |
| | IgA (mg/L) | | Q12 | Q32 | Q34 | Q40 | Follow Up |
| Study Number | Initial | 14 Day | Face Covered | Prior URTI | Passive Smoke | URTI Suspected | Suspected Clinical Cause |
| A01 | 115.5 | 56.1 | N | N | Y | N | Gastro-esophageal reflux |
| A02 | 228.9 | 22.8 | N | Y | Y | Y | RSV+ve Bronchiolitis |
| A03 | 410.6 | 230.9 | N | Y | Y | Y | RSV+ve Bronchiolitis |
| A04 | 91.0 | 28.9 | Y | Y | N | Y | RSV+ve Bronchiolitis |
| A05 | 26.6 | 79.2 | N | Y | N | Y | Reflux with aspiration |

### Salivary Immunoglobulins

Two samples of saliva were collected from each subject. The first sample was collected from the ALTE group within 24 hours of admission to hospital and from the MILD respiratory illness group within 48 hours of presentation of their General Practitioners. The WELL group were recruited from immunisation clinics and saliva collected at ages to approximate the ages of presentation of the ALTE and MILD groups. The second sample was collected 14 days later from each subject.

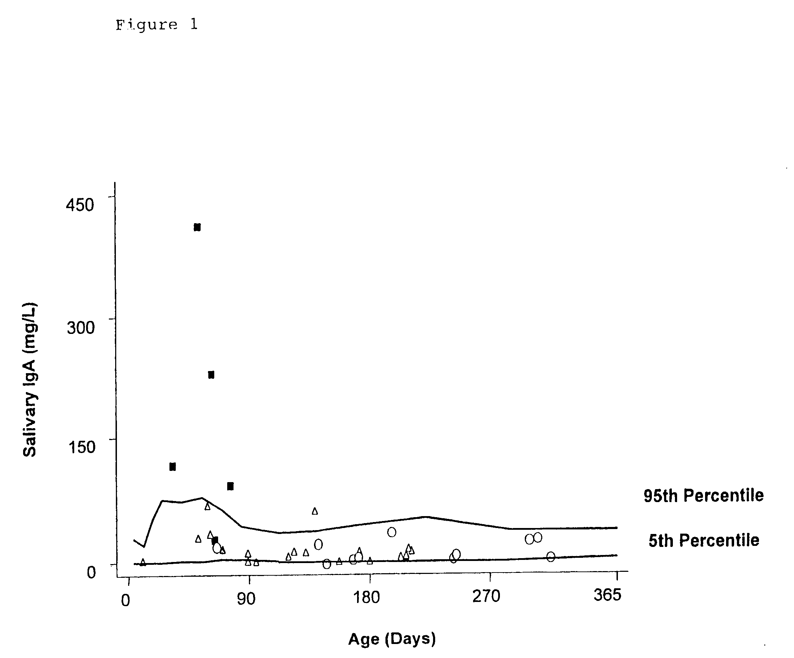

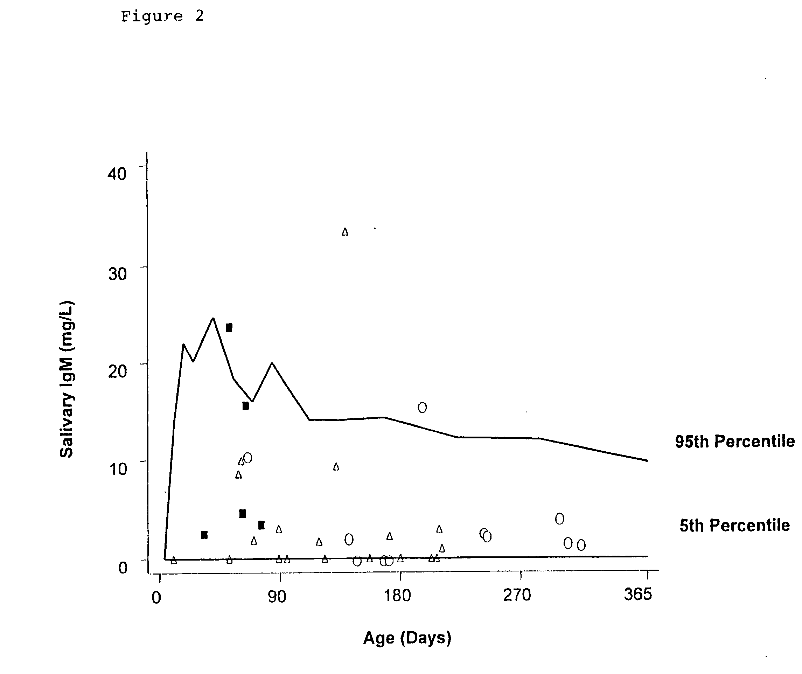

The figures in Appendix C have the age related 5^{th}-95^{th} percentile reference ranges indicated for each salivary immunoglobulin over the first year of life.
• The salivary IgA, IgG and IgM concentration in the ALTE group were all significantly higher than the MILD (Tables 2A and 2B) and WELL (Tables 3A and 3B) groups for both sample 1 and 2 (Figures 1 and 2).
• There were no significant differences between the MILD and WELL groups for either sample 1 or sample 2 (Tables 2C and 3C).
• There were two subjects in the MILD group who had grossly elevated salivary immunoglobulin concentrations in the 14 day collections. (See Appendix C).
• RO3 had an elevated IgA 12 days post infection.
• RO9 had an elevated IgM that is most likely accounted for by immunisation with Triple antigen and *Haemophilus influenzae* B 14 days prior to the saliva collection.

| TABLE 2A | | | | | | | |
|---|---|---|---|---|---|---|---|
| First Sample | | | | | | | |
| Analysis of Immunoglobulins - ALTE vs MILD | | | | | | | |
| | ALTE | | | MILD | | | |
| | N | Median | Range | N | Medial | Range | P-value |
| IgA | 5 | 115.55 | (27-411) | 11 | 9.93 | (0-37) | <0.01 |
| IgG | 5 | 9.21 | (0-16) | 11 | 0.00 | (0-3) | 0.02 |
| IgM | 5 | 4.61 | (3-24) | 11 | 2.18 | (0-16) | 0.04 |

| TABLE 2B | | | | | | | |
|---|---|---|---|---|---|---|---|
| First Sample | | | | | | | |
| Analysis of Immunoglobulins - ALTE vs WELL | | | | | | | |
| | ALTE | | | WELL | | | |
| | N | Median | Range | N | Medial | Range | P-value |
| IgA | 5 | 115.55 | (27-411) | 21 | 11.37 | (0-67) | <0.01 |
| IgG | 5 | 9.21 | (0-16) | 21 | 0.00 | (0-8) | 0.01 |
| IgM | 5 | 4.61 | (3-24) | 21 | 1.00 | (0-33) | 0.01 |

| TABLE 2C | | | | | | | |
|---|---|---|---|---|---|---|---|
| First Sample | | | | | | | |
| Analysis of Immunoglobulins - MILD vs WELL | | | | | | | |
| | MILD | | | WELL | | | |
| | N | Median | Range | N | Medial | Range | P-value |
| IgA | 11 | 9.93 | (0-37) | <0.01 | 11.37 | (0-67) | 0.68 |
| IgG | 11 | 0.00 | (0-3) | 0.02 | 0.00 | (0-8) | 0.66 |
| IgM | 11 | 2.18 | (0-16) | 0.04 | 1.00 | (0-33) | 0.36 |

| TABLE 3A | | | | | | | |
|---|---|---|---|---|---|---|---|
| Second Sample | | | | | | | |
| Analysis of Immunoglobulins - ALTE vs MILD | | | | | | | |
| | ALTE | | | MILD | | | |
| | N | Median | Range | N | Medial | Range | P-value |
| IgA | 5 | 56.06 | (23-231) | 11 | 8.88 | (1-255) | 0.04 |
| IgG | 5 | 2.99 | (2-7) | 11 | 0.00 | (0-4) | 0.03 |
| IgM | 5 | 9.39 | (2-16) | 11 | 2.31 | (0-27) | 0.07 |

| TABLE 3B | | | | | | | |
|---|---|---|---|---|---|---|---|
| Second Sample | | | | | | | |
| Analysis of Immunoglobulins - ALTE vs WELL | | | | | | | |
| | ALTE | | | WELL | | | |
| | N | Median | Range | N | Medial | Range | P-value |
| IgA | 5 | 56.06 | (23-231) | 20 | 10.53 | (0-58) | <0.01 |
| IgG | 5 | 2.99 | (2-7) | 20 | 0.00 | (0-6) | <0.01 |
| IgM | 5 | 9.39 | (2-16) | 20 | 1.66 | (0-14) | <0.0] |

| TABLE 3C | | | | | | | |
|---|---|---|---|---|---|---|---|
| Second Sample | | | | | | | |
| Analysis of Immunoglobulins - ALTE vs WELL | | | | | | | |
| | MILD | | | WELL | | | |
| | N | Median | Range | N | Medial | Range | P-value |
| IgA | 11 | 8.88 | (1-255) | 20 | 10.53 | (0-58) | 0.71 |
| IgG | 11 | 0.00 | (0-4) | 20 | 0.00 | (0-6) | 0.75 |
| IgM | 11 | 2.31 | (0-27) | 20 | 1.66 | (0-14) | 0.56 |

### Conclusions

- The grossly elevated salivary IgA concentration in 4 of 5 ALTE subjects at presentation was not observed in the MILD or WELL control groups. Salivary IgA can therefore act as a marker for ALTE (and SIDS) in subjects presenting with an otherwise mild respiratory illness. This was an unexpected result since the prior art [1] suggested IgM would be the most useful parameter in prediction of ALTE/SIDS susceptibility.
- The elevated salivary IgA and IgM concentrations in 4 of ALTE support the concept of an infection or inflammatory cause in ALTE (and SIDS).
- RSV positive Bronchiolitis was evident in 3 of 5 ALTE subjects.

### EXAMPLE 2

### Total IgA and IgA1 subclass in Saliva of Infants with ALTE

### Study Groups

Saliva samples were collected from infants on the day of admission to hospital for an unexplained acute life-threatening episode (ALTE). The infants were included in this study if all congenital or obstructive causes of apnoea had been excluded. This group of subjects have been classified as the "near-miss SIDS" infants.

Saliva was collected from age matched control subjects in two categories. Normal healthy infants were recruited from the Child Immunisation Clinics and classified as WELL infants. The second group was recruited from general practitioners, who referred infants with a mild upper respiratory infection and these infants were classified as the MILD infection control group. Saliva was collected on the day of referral with the mild infection.

### Laboratory Analysis

Saliva samples were assayed by an Enzyme Linked Immunosorbant Assay (ELISA) to detect total IgA and IgA1 subclass antibodies. The assay uses a WHO/IUIS approved monoclonal antibody for IgA1 subclass as the capture antibody in conjunction with a polyclonal antibody-enzyme labelled detection system.

### Results

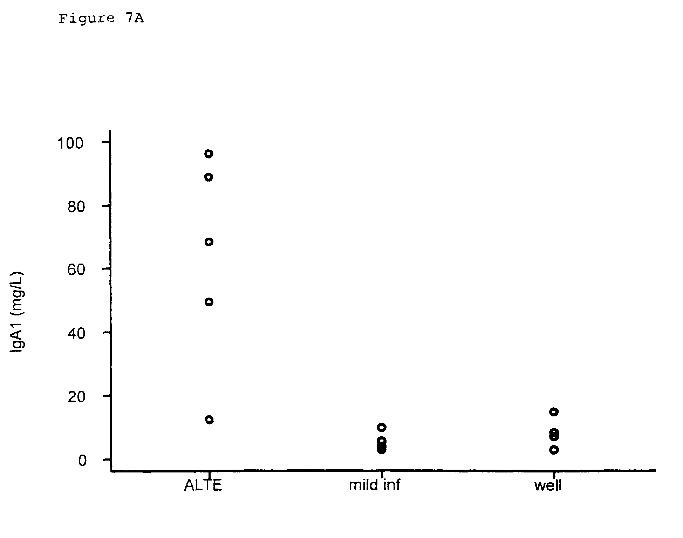

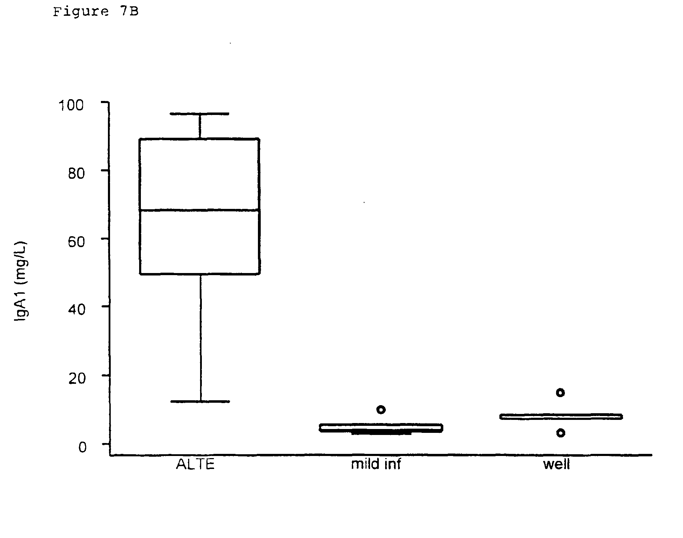

The results indicate that the concentrations of IgA1 subclass in the saliva from infants suffering an ALTE were significantly higher than the concentrations for the infants in the control groups of normal healthy infants and those suffering a mild upper respiratory infection (p=0.009) (Table 4 and Figures 7A and 7B).

The concentrations of IgA1 in saliva from the normal healthy infants were not significantly different from those with mild respiratory illnesses.

Five samples were assayed from each of the three study groups: ALTE babies, babies with mild infection, and well babies. The level of IgA1 was generally much higher in the samples from the ALTE babies compared to the levels in the other two groups (p=0.009). Levels in the mild infection and well baby groups were similar.

### Statistical Analysis

The non-parametric Kruskal-Wallis test was used to compare the distributions of IgA1 values for the three groups. The probability of the three sample groups having equal Ig.A1 distributions is p=0.009. Due to small sample sizes, the estimated 95% confidence intervals about the group medians are equivalent to the range (ie. min, max) of the data.

**Table 1**

| | IgA1 (mg/L) | | |
|---|---|---|---|
| *group* | min | max | median |
| ALTE | 12.27 | 96.35 | 68.35 |
| Mild infection | 2.79 | 9.78 | 9.92 |
| Well | 3.02 | 14.85 | 7.29 |

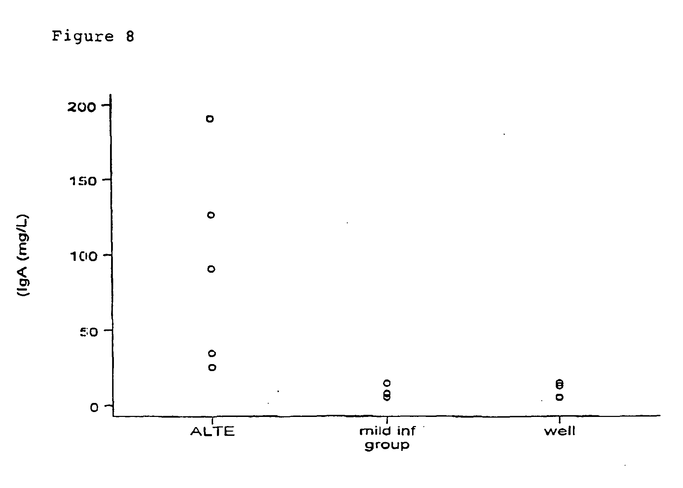

Total IgA levels were also elevated in the same infants who took part in this study (Figure 8).

### Conclusion

IgA1 concentrations are significantly elevated in infants suffering an unexplained ALTE (4 out of 5 children). Three out of the same 5 children with ALTE were found to have elevated total IgA levels. Therefore, although it is clear that both IgA and IgA1 are useful parameters in the prediction of ALTE, IgA1 levels may be the more useful parameter. Since ALTE are classified as "near-miss" SIDS (when no other medical condition is identified), it follows that both IgA and IgA1 are also useful parameters in the prediction of SIDS.

Although the invention has been described with reference to specific examples, it will be appreciated by those skilled in the art that the invention may be embodied in many other forms.

### REFERENCES

1. Gleeson M. Clancy RL, Cripps AW (1993) Mucosal immune response in a case of sudden infant death syndrome. Paed Res Vol 33:No 6 554-556.
2. Thrane PS, Rognum TO, Brandtzaeg P (1990) Increased immune response in upper respiratory and digestive tracts in SIDS. Lancet 1:229-230.
3. Forsyth KD, Weeks SC, Koh L. Skinner J, Bradley J (1989) Lung immunoglobulins in the sudden infant death syndrome. British Medical Journal 298:23-26.
4. Stolenberg L. Saugstad OD, Rognum TO (1992) Sudden infant death syndrome victims show local immunoglobulin M response in tracheal wall and immunoglobulin. A response in duodenal mucosa. Paed Res 31:372-375.
5. Guntheroth WG (1989) lnterleukin-1 as intermediary causing prolonged sleep apnea and SIDS during respiratory infections. Med Hypotheses 28:121-123^{.}
6. Gleeson M, Dobson AJ, Firman DW. Cripps AW. Clancy RL, Wlodarczyk JH, Hensley MJ (1991) The variability of immunoglobulins and albumin in salivary secretions of children. Scand J Immunol. 33: 533-541.
7. Wilkinson M, Jones C (1997) Australian SIDS statistics. Australia SIDS Conference. Abstract 301.
8. Blackwell CC, Weir DM, Busuttil A (1997) Infectious Agents and SIDS: Analysis of risk factors and preventive measures. SIDS and Infant Mortality. (In press).
9. Byart R (1997) Does SIDS exist anymore? Australian SIDS Conference. Directions of SIDS Research, Presentation.
10. Shannon DC, Kelly DH (1982) SIDS and near-SIDS. N. Engl J Med 306:959-965.
11. Hoffman JH, Damus K, Hillman L, Krongrad E (1988) Risk factors for SIDS. Ann NY Acad Sci 533:13-20.
12. Nelson EA, Taylor BJ, Mackay SC (1989) Childcare practices and the sudden infant death syndrome. Aust Paed J 25:202-204.
13. Naeye RL (1990) Preventing the sudden infant death syndrome. Paediatr Perinat Epidemio 4:12:21.
14. Ogra PL, Ogra SS. Coppola PR (1975) Secretory component and sudden infant death syndrome. Lancet 2:387-390.
15. Ostergaard PA, Blom M (1977) Whole saliva immunoglobulin levels in 60 healthy children determined by a sensitive electroimmuno technique after prior carbamylation. Jnr Clin Chem Clin Biochem 15:393-6.
16. Gleeson M, McDonald WA, Cripps AW, Pyne DB, Clancy RL, Fricker PA, Wlodarczyk JH (1995) Exercise, stress, and mucosal immunity in elite swimmers. In Advances in Mucosal Immunology. Ed J. Mestecky et al. Plenum Press New York 571-574.

## Claims

1. A method of assessing potential susceptibility to development of ALTE (acute life threatening episode) and/or SIDS (sudden infant death syndrome) in a subject including:
(a) determination of the immunoglobulin A (IgA) level in a sample from the subject; and
(b) prediction of susceptibility to development of ALTE and/or SIDS by comparison of said IgA level with a predetermined standard.

2. A method of assessing potential susceptibility to development of ALTE and/or SIDS in a subject including:
(a) determination of immunoglobulin A1 (IgA1) level in a sample from the subject; and
(b) prediction of susceptibility to development of ALTE and/or SIDS by comparison of said IgA1 level with a predetermined standard.

3. A method according to claim 1 or claim 2 wherein the subject is a human infant.

4. A method according to any one of claims 1 to 3 wherein the sample is a sample from a subject at the time of, or any time up to approximately 2 weeks after, an upper respiratory tract infection (URTI) and/or symptoms.

5. A method according to any one of claims 1 to 4 wherein the immunoglobulin is secretory immunoglobulin.

6. A method according to claim 5 wherein the secretory immunoglobulin is salivary immunoglobulin.

7. A method according to claim 6 wherein the sample is whole unstimulated saliva.

8. A method according to any one of claims 1 to 7 wherein the subject is not fasting when the sample is collected.

9. A method according to any one of claims 1 to 8 wherein the immunoglobulin level is determined by ELISA.

10. A method according to any one of claims 1 to 8 wherein the immunoglobulin level is determined by radial immunodiffusion.

11. A method according to any one of clams 1 to 9 wherein the immunoglobulin level is analysed by a rapid near-subject assay.

12. A method according to any one of claims 1 to 11 wherein the immunoglobulin level is determined by contacting a body secretion with an assay device or system on a support.

13. A method according to any one of claims 1 to 12 wherein the standard is a normal population standard.

14. A method according to any one of claims 1 to 12 wherein the standard is an internal personal standard.

15. A method according to any one of claims 1 to 14 further including comparison of the ratio of immunoglobulin level to other indices.

16. A method according to claim 15 wherein the other indices are selected from the group consisting of IgM, IgG, acute phase reactants or cellular components.

## Patentansprüche

1. Verfahren zur Beurteilung einer potentiellen Anfälligkeit gegenüber der Entwicklung von ALTE (akutes lebensbedrohliches Ereignis) und/oder SIDS (plötzlicher Kindstod) bei einem Patienten, das die folgenden Schritte beinhaltet:
(a) Bestimmen des Immunglobulin-A-(IgA)-Wertes in einer Probe von dem Patienten; und
(b) Vorhersagen der Anfälligkeit gegenüber der Entwicklung von ALTE und/oder SIDS durch einen Vergleich des genannten IgA-Wertes mit einem vorbestimmten Standard.

2. Verfahren zur Beurteilung der potentiellen Anfälligkeit gegenüber der Entwicklung von ALTE und/oder SIDS bei einem Patienten, das die folgenden Schritte beinhaltet:
(a) Bestimmen des Immunglobulin-A1-(IgAl)-Wertes in einer Probe von dem Patienten; und
(b) Vorhersagen der Anfälligkeit gegenüber der Entwicklung von ALTE und/oder SIDS durch einen Vergleich des genannten IgAl-Wertes mit einem vorbestimmten Standard.

3. Verfahren nach Anspruch 1 oder Anspruch 2, wobei der Patient ein menschlicher Säugling ist.

4. Verfahren nach einem der Ansprüche 1 bis 3, wobei die Probe eine Probe von einem Patienten zum Zeitpunkt von oder zu einem beliebigen Zeitpunkt bis zu etwa 2 Wochen nach einer Infektion der oberen Atemwege (URTI) und/oder Symptomen ist.

5. Verfahren nach einem der Ansprüche 1 bis 4, wobei das Immunglobulin sekretorisches Immunglobulin ist.

6. Verfahren nach Anspruch 5, wobei das sekretorische Immunglobulin Speichelimmunglobulin ist.

7. Verfahren nach Anspruch 6, wobei die Probe unstimulierter Vollspeichel ist.

8. Verfahren nach einem der Ansprüche 1 bis 7, wobei der Patient nicht nüchtern ist, wenn die Probe genommen wird.

9. Verfahren nach einem der Ansprüche 1 bis 8, wobei der Immunglobulinwert durch ELISA ermittelt wird.

10. Verfahren nach einem der Ansprüche 1 bis 8, wobei der Immunglobulinwert durch radiale Immundiffusion ermittelt wird.

11. Verfahren nach einem der Ansprüche 1 bis 9, wobei der Immunglobulinwert durch einen schnellen, patientennahen Test analysiert wird.

12. Verfahren nach einem der Ansprüche 1 bis 11, wobei der Immunglobulinwert durch Inkontaktbringen eines Körpersekrets mit einer/einem Testvorrichtung oder -system auf einem Träger ermittelt wird.

13. Verfahren nach einem der Ansprüche 1 bis 12, wobei der Standard ein normaler Populationsstandard ist.

14. Verfahren nach einem der Ansprüche 1 bis 12, wobei der Standard ein innerer persönlicher Standard ist.

15. Verfahren nach einem der Ansprüche 1 bis 14, das ferner einen Vergleich des Verhältnisses zwischen Immunglobulinwert und anderen Indizes beinhaltet.

16. Verfahren nach Anspruch 15, wobei die anderen Indizes ausgewählt sind aus der Gruppe bestehend aus IgM, IgG, Akutphasenreaktanten oder Zellkomponenten.

## Revendications

1. Procédé d'évaluation de la susceptibilité potentielle de développement d'un épisode aigu à risque mortel (ALTE) et/ou du syndrome de la mort subite du nourrisson (MSN) chez un sujet, comprenant:
(a) déterminer le taux d'immunoglobuline A (IgA) dans un échantillon du sujet;
(b) prévoir la susceptibilité de développement d'ALTE et/ou de MSN en comparant ledit taux d'IgA à un étalon prédéterminé.

2. Procédé d'évaluation de la susceptibilité potentielle de développement d'ALTE et/ou de MSN chez un sujet, comprenant:
(a) déterminer le taux d'immunoglobuline A1 (IgA1) dans un échantillon du sujet;
(b) prévoir la susceptibilité de développement d'ALTE et/ou de MSN en comparant ledit taux d'IgA1 à un étalon prédéterminé.

3. Procédé selon la revendication 1 ou la revendication 2, dans lequel le sujet est un nourrisson humain.

4. Procédé selon l'une quelconque des revendications 1 à 3, dans lequel l'échantillon est un échantillon d'un sujet au moment de, ou à n'importe quel moment approximativement deux semaines après une infection des voies respiratoires supérieures (IVRS) et/ou de symptômes.

5. Procédé selon l'une quelconque des revendications 1 à 4, dans lequel l'immunoglobuline est une immunoglobuline sécrétoire.

6. Procédé selon la revendication 5, dans lequel l'immunoglobuline sécrétoire est une immunoglobuline salivaire.

7. Procédé selon la revendication 6, dans lequel l'échantillon est de la salive complète non stimulée.

8. Procédé selon l'une quelconque des revendications 1 à 7, dans lequel le sujet n'est pas à jeun lorsque l'échantillon est recueilli.

9. Procédé selon l'une quelconque des revendications 1 à 8, dans lequel le taux d'immunoglobuline est déterminé par ELISA.

10. Procédé selon l'une quelconque des revendications 1 à 8, dans lequel le taux d'immunoglobuline est déterminé par immunodiffusion radiale.

11. Procédé selon l'une quelconque des revendications 1 à 9, dans lequel le taux d'immunoglobuline est analysé par une analyse rapide au chevet du sujet.

12. Procédé selon l'une quelconque des revendications 1 à 11, dans lequel le taux d'immunoglobuline est déterminé en mettant une sécrétion corporelle en contact avec un dispositif ou un système d'essai sur un support.

13. Procédé selon l'une quelconque des revendications 1 à 12, dans lequel l'étalon est un étalon de population normale.

14. Procédé selon l'une quelconque des revendications 1 à 12, dans lequel l'étalon est un étalon personnel interne.

15. Procédé selon l'une quelconque des revendications 1 à 14, comprenant en outre une comparaison du rapport du taux d'immunoglobuline à d'autres indices.

16. Procédé selon la revendication 15, dans lequel les autres indices sont sélectionnés parmi le groupe consistant en IgM, IgG, réactants en phase aiguë ou composants cellulaires.
